# EUROPEAN PATENT SPECIFICATION

(11) **EP 0 407 551 B1**
(45) Date of publication and mention of the grant of the patent: **26.04.1995**
(21) Application number: 90902415.0
(22) Date of filing: 11.01.1990
(51) Int. Cl.: A61F 6/04, B65D 27/00

(54) **A DEVICE FOR WRAPPED CONDOMS**
ANWENDUNGSVORRICHTUNG FÜR VERPACKTE, AUFGEROLLTE KONDOME
DISPOSITIF D'APPLICATION POUR PRESERVATIFS EMBALLES ENROULES

(30) Priority: 31.01.1989 SE 8900323
(43) Date of publication of application: 16.01.1991
(73) Proprietor: HERS PRODUKTER, S-688 00 Storfors (SE)
(72) Inventor: BACKMAN, Hans, S-681 00 Kristinehamn (SE)
(74) Representative: Lautmann, Kurt O.
(86) International application number: SE9000020
(87) International publication number: WO9008522

(56) References cited:
- SE-B- 0 382 156

## Description

The present invention relates to a wrapped condom according to the preamble of claim 1. The wrapping is generally rectangular in shape and is formed of two layers of a suitable material plated one on top of the other and joined together around the edges. When the condom is to be used the wrapping is normally torn in two straight across its longitudinal side. The condom is then applied by unrolling it with the fingers. Prior to application the condom is in fully rolled state. Certainly difficulties arise in applying the condom since it may be slippery and there is a risk of puncturing the condom with fingernails.

Such a device is disclosed in DE-A-2410697 (SE-B-382156).

The object of the present invention is to eliminate the above-mentioned drawbacks. This is achieved by the characterizing portion of claim 1. The condom with its tape ends is placed in its envelope or wrapping. The free tape ends are suitably secured to the envelope in such a manner that when the envelope is opened and forms two parts, the two ends of the tapes are attached one to each half of the envelope. The two parts of the envelope then form tabs which can be gripped. When the condom is applied these tabs are then pulled and the condom will be unrolled without the fingers having to come into contact with the condom itself.

An embodiment of the invention will be described with reference to the accompanying drawings in which
- Figure 1: shows a condom envelope being broken open,
- Figure 2: shows the initial stage of applying the condom,
- Figure 3: shows the condom applied and partially unrolled, and
- Figure 4: shows the condom fully applied.

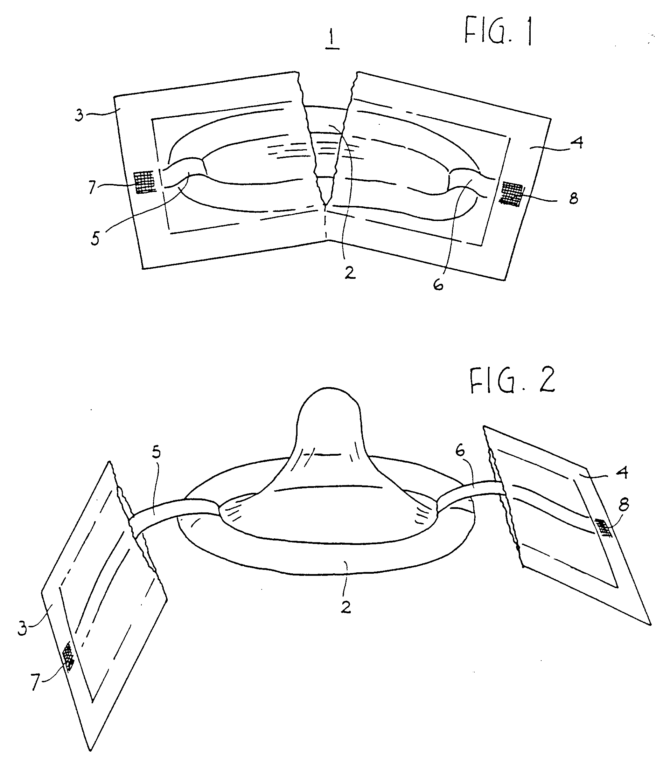

Figure 1 shows an envelope or wrapping containing a condom 2. The envelope 1 is in the process of being torn open to form two parts 3 and 4. In rolled-up state the outer ends of the two tapes 5 and 6 are exposed. One end 7 of the tape 5 is attached to the part 3 of the envelope and one end 8 of the tape 6 is attached to the part 4 of the envelope.

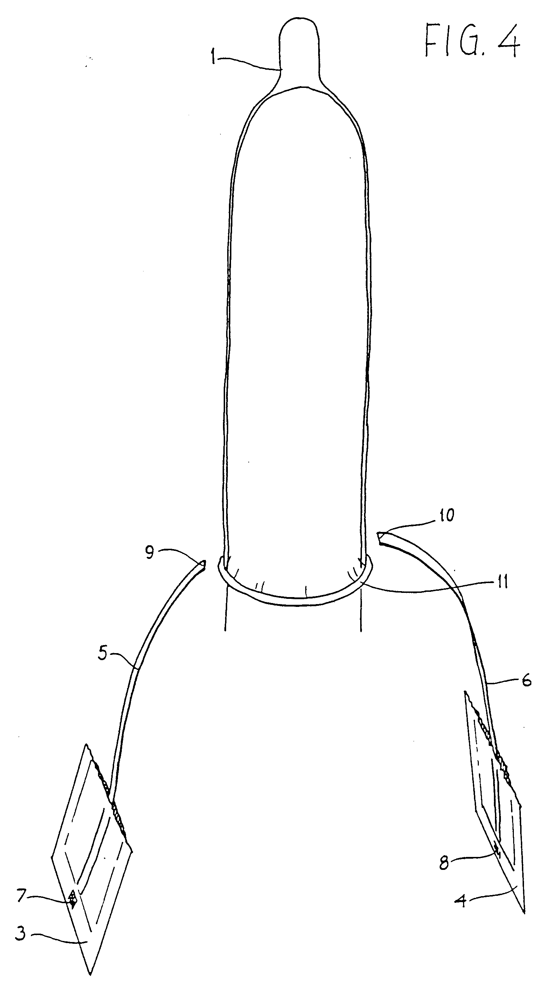

Figure 2 shows the condom when its envelope 1 has been fully opened. The condom is now ready to be applied. Prior to rolling the condom 2, the inner ends 9 and 10 (Fig. 4) have been arranged in some suitable manner at the rim of the condom. The tape ends 9 and 10 may be welded to said rim. The two tapes 5 and 6 are rolled up at the same time as the condom itself and, prior to use, the tapes are in the positions shown in Figures 1 and 2.

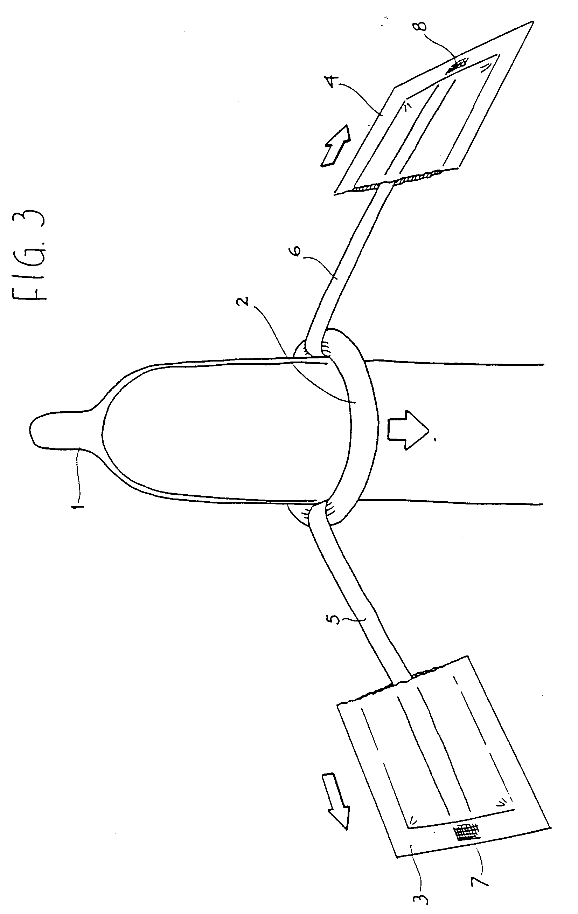

When the condom is to be applied (Fig. 3) it is correctly placed and the two parts 3 and 4 of the envelope gripped, whereupon the condom will be automatically unrolled without having to be unrolled with only the assistance of the tapes. The two parts 3 and 4 of the envelope offer excellent gripping tabs.

The arrangement of two tapes 5 and 6 diametrically opposite each other at the open end of a condom greatly facilitates applying and unrolling the condom.

## Claims

1. A device for wrapped condoms (2), the condom (2) being in rolled state and surrounded by an envelope (1), whereby the condom on its outside has two tapes, threads or the like, which are longer than the condom, which are diametrically opposite located in relation to each other and which have one end fixed at the opening end of the condom whereby the other ends of the condom are protruding the condom in rolled state,
**characterized** in that the envelope (1) can be torn off in two predestinated parts (3, 4) which preferably are of the same size, which are opposite each other, which are designed to form tabs for gripping and which are connected with each of the said other ends.

## Patentansprüche

1. Eine Vorrichtung für verpackte Kondome (2), das Kondom (2) in zusammengerolltem Zustand und von einer Umhüllung (1) umgeben, wodurch das Kondom an seiner Außenseite zwei Streifen, Fäden oder dergleichen hat, die länger als das Kondom sind, die im Verhältnis zueinander diametral angeordnet sind, und die mit einem Ende am offenen Ende des Kondoms befestigt sind, wodurch die anderen Enden des Kondoms aus dem Kondom in zusammengerolltem Zustand herausragen, dadurch **gekennzeicnet,** daß die Umhüllung (1) in zwei im voraus festgelegte Teile (3, 4) aufgerissen werden kann, die vorzugsweise von gleicher Größe sind und die mittig voreinander liegen und so ausgelegt sind, daß sie Laschen zum Fügen bilden, die mit jedem der genannten anderen Enden verbunden sind.

## Revendications

1. Un dispositif pour préservatifs masculins (2) emballés, le préservatif (2) étant en un état roulé et entouré d'une enveloppe (1), le préservatif ayant à son extérieur deux bandes, fils ou similaire oui sont plus longs que le préservatif, localisés diamétralement opposés l'un par rapport à l'autre et dont un bout est fixé au bout d'ouverture du préservatif, les autres bouts du préservatif étant en saillie du préservatif en état roulé, **caractérisé** en ce que ladite enveloppe (1) peut être déchirée en deux parties prédestinées (3, 4) de préférence de la même taille, étant opposées l'une par rapport à l'autre et conçues pour former des languettes de prise et oui sont connectées avec chacun desdits autres bouts.
